# EUROPÄISCHE PATENTANMELDUNG

(11) **EP 1 938 860 A1**
(43) Veröffentlichungstag der Anmeldung: **02.07.2008**
(21) Anmeldenummer: 07033576.5
(22) Anmeldetag: 06.12.2007
(51) Int. Cl.: A61N 1/32, A61N 1/36, A61B 5/11, A61N 1/04

(54) **Vorrichtung zur transkutanen elektrischen Stimulation motorischer und / oder sensorischer Nerven**

(30) Priorität: 11.12.2006 DE 102006058346
(71) Anmelder: Lohmann & Rauscher GmbH, 2525 Schönau/Triesting (AT)
(72) Erfinder: Popovic, Dejan, 11000 Belgrad (RS); Rohrer, Christian, 4020 Linz (AT)
(74) Vertreter: Leinweber & Zimmermann

(57) **Zusammenfassung**

Die Erfindung betrifft eine Vorrichtung zur transkutanen elektrischen Stimulation motorischer und/oder sensorischer Nerven mit einer bspw. im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung, wobei der Stimulationseinrichtung mindestens eine zum Erfassen einer Körperbewegung betreibbare Bewegungssensoranordnung zugeordnet ist und die Stimulationseinrichtung zum Anlegen des elektrischen Stroms in Abhängigkeit von von der Bewegungssensoranordnung abgegebenen Bewegungssignalen betreibbar ist.

## Beschreibung

Die Erfindung betrifft eine Vorrichtung zur transkutanen elektrischen Stimulation motorischer und/oder sensorischer Nerven mit bspw. einer im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung sowie ein Verfahren zur transkutanen elektrischen Stimulation motorischer und/oder sensorischer Nerven mit einer solchen Vorrichtung.

Vorrichtungen der eingangs beschriebenen Art werden bspw. zum gezielten Aufbau der Rücken- und/oder Bauchmuskulatur eingesetzt. Dadurch kann u.a. die Wirbelsäule im Lendenwirbelbereich gestützt werden. Dabei wird üblicherweise zwischen der Elektrodenanordnung und der Haut ein leitfähiges Gel angeordnet, um die eine Muskelaktivierung verursachende Stimulation der motorischen Nerven mit Hilfe des angelegten elektrischen Stroms zu verbessern. Dabei kann es bei der selektiven Einleitung des elektrischen Stroms in den Bereich der motorischen Nerven zu Problemen kommen, wenn das leitfähige Gel über eine zu große Fläche verteilt wird. Angesichts dieses Mangels wird in der US 4,919,148 eine Verbesserung bekannter Vorrichtungen der eingangs beschriebenen Art vorgeschlagen, mit der die Ausbreitung des leitfähigen Gels mit Hilfe einer die Elektrodenanordnung tragenden textilen Struktur gehemmt wird. Weitere Vorrichtungen zur transkutanen und/oder perkutanen elektrischen Stimulation motorischer und/oder sensorischer Nerven sind bspw. in der US 6,445,955 B1, CA 2 496 879 A1, DE 103 00 069 A1, DE 102 48 235 A1, DE 101 55 551 A1, EP 0 052 087 A1, DE 601 11 388 T2, EP 1 144 045 B1, DE 295 22 012 U1 und der DE 202 00 685 U1 beschrieben. Darüber hinaus ist auch schon vorgeschlagen worden, die Nervenstimulation in Abhängigkeit von der Körperhaltung und/oder der bspw. elektromyographisch bestimmten Muskelaktivität zu steuern. Entsprechende Vorrichtungen sind in der EP 1121 956 A1, US 6,341,237 B1, US 5,643,329, DE 35 16 279 A1 und der DE 20 2006 008 616 U1 angegeben. Endlich ist in der EP 1 324 403 B1 auch noch der Einsatz von elektroaktiven elastischen Aktuatoren zur Applikation von Bewegungen des menschlichen Körpers beschrieben.

Allerdings hat es sich auch beim Einsatz der in den genannten Schriften beschriebenen Vorrichtungen gezeigt, daß es trotz der damit aufgebauten Muskulatur häufig zu Schmerzen im Lendenwirbelbereich kommt.

Angesichts dieser Probleme im Stand der Technik liegt der Erfindung die Aufgabe zugrunde, eine Vorrichtung der eingangs genannten Art bereitzustellen, mit der ein Muskelaufbau unter gleichzeitiger Förderung schmerzfreier bzw. unschädlicher Bewegungen möglich ist.

Gemäß einem ersten Gesichtspunkt der Erfindung wird diese Aufgabe durch eine Weiterbildung der bekannten Vorrichtungen gelöst, die im wesentlichen dadurch gekennzeichnet ist, daß der Stimulationseinrichtung mindestens eine zum Erfassen einer Körperbewegung betreibbare Bewegungssensoranordnung zugeordnet ist und die Stimulationseinrichtung zum Anlegen des elektrischen Stroms in Abhängigkeit von von der Bewegungssensoranordnung abgegebenen Bewegungssignalen betreibbar ist. Dabei kann die Bewegungssensoranordnung an den Körper anlegbare Sensoren aufweisen, die räumlich getrennt von den Elektroden der Elektrodenanordnung am Körper angeordnet sein können. Vorstellbar ist, daß die Bewegungssensoren am Bauch und die Elektroden am Rücken angeordnet sind.

Diese Erfindung geht auf die Erkenntnis zurück, daß die beim Einsatz der bekannten Vorrichtungen beobachteten Probleme in erster Linie darauf zurückzuführen sind, daß die elektrische Stimulation motorischer Nerven auch bei regionaler Eingrenzung der Einleitung des elektrischen Stroms in den menschlichen Körper zu Fehlbewegungen führen kann, welche schmerzhafte Zustände im Lendenwirbelbereich zur Folge haben können, wobei dieser Mangel auch nicht durch eine Stimulation in Abhängigkeit von der mit geeigneten Sensoren erfaßten Muskelaktivität oder der Körperhaltung beseitigt werden kann. Erfindungsgemäß wird diese unerwünschte Folge dadurch vermieden, daß nicht nur die Muskelaktivität oder Körperhaltung, sondern auch die Körperbewegung mit Hilfe einer geeigneten Bewegungssensoranordnung überwacht wird und bei ungünstigen oder schädlichen Bewegungen eine vorgegebene Stromeinleitung erfolgt. Dabei kann durch die vorgegebene Stromeinleitung entweder unmittelbar die Muskulatur des menschlichen Körpers so aktiviert werden, daß automatisch der ungünstigen Bewegung entgegengewirkt wird, oder es kann durch die Stromeinleitung ein ggf. nur unterbewußt wahrnehmbares Signal gesetzt werden, mit dem der Benutzer einer erfindungsgemäßen Vorrichtung dazu veranlaßt wird, die ungünstige Bewegung zu beenden.

Erstaunlicherweise hat sich in beiden Fällen gezeigt, daß sich bei regelmäßigem Einsatz erfindungsgemäßer Vorrichtungen ein Lerneffekt einstellt, der zur Folge hat, daß die Schmerzzustände verursachenden Bewegungen dauerhaft unterlassen werden.

Zweckmäßigerweise weist die Stimulationseinrichtung einer erfindungsgemäßen Vorrichtung eine Speicheranordnung auf, in der vorgegebene Bewegungsmuster darstellende Daten abgelegt sind. Dann können die mit der Bewegungssensoranordnung erfaßten Körperbewegungen mit den vorgegebenen Bewegungsmustern verglichen und bei Abweichungen von vorgegebenen Bewegungsmustern kann durch entsprechende Ansteuerung der Elektrodenanordnung die Muskelaktivität so beeinflußt werden, daß das vorgegebene Bewegungsmuster wieder erreicht wird.

Die Bewegungssensoranordnung kann ein, zwei oder mehr auf der Körperoberfläche angeordnete Sensorelemente aufweisen, wobei mit diesem Sensorsystem vorzugsweise für Rumpfrotationen bei unbewegten Beinen, Rumpfbeugen in einer physiologisch bedenklichen Weise, das Beugen auf eine Seite od. dgl., charakteristische Änderungen nachgewiesen werden können. Mit dem Sensorsystem können zweckmäßigerweise auch Änderungsgeschwindigkeiten bezüglich der Entfernung und/oder Ausrichtung zwischen zwei Sensorelementen auf der Körperoberfläche nachgewiesen werden. Bei einer zweckmäßigen Ausführungsform der Erfindung weist das Sensorsystem zwei Festkörper-Axialbeschleunigungsmeßgeräte auf. Es ist allerdings nicht auf diese Technologie begrenzt, sondern kann auch optische Fasern, optische Sender, Ultraschallsender, flexible Substrate mit Meßgeräten für mechanische Spannungen oder andere Meßgeräte aufweisen. Im Rahmen der Erfindung einsetzbare Bewegungssensoranordnungen sind bspw. in der DE 42 05 790 A1 beschrieben. Der Offenbarungsgehalt dieser Schrift hinsichtlich des Aufbaus und der Funktion von Bewegungssensoranordnungen zum Erfassen der Bewegung des menschlichen Körpers wird hiermit durch ausdrückliche Inbezugnahme in diese Beschreibung aufgenommen.

Der Einsatz einer Bewegungssensoranordnung hat sich im Rahmen dieser Erfindung auch deswegen als besonders sinnvoll erwiesen, weil sich der zeitliche Ablauf und das Niveau der Muskelaktivierung zum Erhalt des gewünschten Erfolgs zeitlich und während einer Bewegung ändern kann.

Gemäß einem weiteren Gesichtspunkt der Erfindung weist eine erfindungsgemäße Vorrichtung zur transkutanen elektrischen Stimulation motorischer Nerven mit einer bspw. im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung eine Elektrodenanordnung mit vier oder mehr unabhängig voneinander mit elektrischem Strom beaufschlagbaren Elektrodenpaaren auf. Mit einer solchen Anordnung lassen sich vier oder mehr Muskelgruppen unabhängig voneinander aktivieren, so daß vorgegebene Bewegungsmuster besonders genau nachvollzogen werden können. Mit vier oder mehr unabhängig voneinander ansteuerbaren Elektrodenpaaren können zwei oder mehr Paare zusammengehöriger Muskelgruppen (z.B. Agonist und Antagonist) stimuliert werden, um so funktionale Bewegungen besonders gut nachbilden zu können. Die so erreichte Funktion ist ein sehr wichtiges Element bei der Rehabilitation, weil sie zu einer schnelleren und effektiveren kortikalen Neuorganisation führt. Dabei hat es sich als besonders zweckmäßig erwiesen, wenn die Elektrodenanordnung sechs oder mehr, insbes. acht oder mehr, unabhängig voneinander mit elektrischem Strom beaufschlagbare Elektrodenpaare aufweist. Dabei kann die Stimulationseinrichtung eine Steuereinrichtung aufweisen, mit der die einzelnen Elektrodenpaare unabhängig voneinander angesteuert werden. Diese Steuereinrichtung kann die Elektrodenpaare vorzugsweise in Abhängigkeit von den mit der Bewegungssensoranordnung erzeugten Bewegungssignalen ansteuern. Zusätzlich oder alternativ ist aber auch an solche Vorrichtungen gedacht, bei denen die Elektroden gemäß vorgegebener Programme derart angesteuert werden, daß die vorgegebenen Bewegungsmuster nachvollzogen werden. Im Rahmen der Erfindung ist es besonders zweckmäßig und zum Erhalt der vorgegebenen Bewegungsmuster bevorzugt, wenn die Elektrodenpaare hinsichtlich der Stimulationsfrequenz und/oder der Stimulationsamplitude unabhängig voneinander ansteuerbar sind.

Gemäß einem dritten Gesichtspunkt der Erfindung weist die Elektrodenanordnung mindestens ein Elektrodenpaar mit einer zum Einleiten des elektrischen Stroms in den menschlichen Körper über eine erste Kontaktfläche ausgelegten ersten Elektrode und einer zum Ableiten des elektrischen Stroms aus dem menschlichen Körper über eine zweite Kontaktfläche ausgelegten zweiten Elektrode auf, wobei die Größe, Form und/oder Position mindestens einer Kontaktfläche nach Anlegen des Elektrodenpaares an den menschlichen Körper veränderbar ist. Mit einer solchen Elektrodenanordnung kann die Einleitung der die Muskelaktivierung veranlassenden Stromimpulse besonders genau an die Besonderheiten der Anatomie des Benutzers einer erfindungsgemäßen Vorrichtung angepaßt werden, wenn damit die Größe, Form und Position der Kontaktfläche mindestens einer Elektrode so angepaßt wird, daß die Stromeinleitung genau oberhalb der zu stimulierenden Nerven erfolgt. Ferner kann über die Einstellung der Größe der Kontaktfläche auch die Stromstärke der eingeleiteten Stromimpulse eingestellt werden. Diese Einstellungen können bei einer erfindungsgemäßen Vorrichtung nach Anlegen der Elektrodenanordnung an den menschlichen Körper erfolgen, so daß auf ein lästiges Ablösen und erneutes Anbringen einzelner Elektroden verzichtet werden kann.

Die Anpassung der Größe, Form und Position der Kontaktflächen der Elektroden an die Besonderheiten der Anatomie des Benutzers erfindungsgemäßer Vorrichtungen kann besonders einfach erfolgen, wenn mindestens eine Elektrode ein Elektrodenfeld mit einer Anzahl von elektrisch voneinander isolierten und einzeln oder in Gruppen getrennt voneinander ansteuerbaren Elektrodenelementen mit einer jeweiligen Kontaktflächengröße von 1 cm² oder mehr aufweist. Mit einer solchen als Elektrodenfeld ausgeführten Elektrode kann die Einleitung der die Muskelaktivierung veranlassenden Stromimpulse besonders genau an die Besonderheiten der Anatomie des Benutzers einer erfindungsgemäßen Vorrichtung angepaßt werden, wenn damit nur die Elektrodenelemente angesteuert werden, welche unmittelbar oberhalb der zu stimulierenden Nerven angeordnet sind. Dabei kann mit der als Elektrodenfeld ausgeführten Elektrode die Größe und Form der aktivierten Elektrode ebenso eingestellt werden wie die Stromstärke der eingeleiteten Stromimpulse, indem die Elektrodenelemente entsprechend angesteuert werden.

Die Elektrodenelemente des Elektrodenfeldes können nach Art einer Matrix, insbes. in Form einer Rechteckmatrix, mit einer Mehrzahl von parallel zueinander verlaufenden Elektrodenelementreihen und einer Mehrzahl von senkrecht zu den Elektrodenelementreihen verlaufenden Elektrodenelementspalten angeordnet sein.

Neben der Rechteckform können im Rahmen der Erfindung aber auch andere Formen, wie etwa die Form eines Sechsecks, Kreises o. dgl., gewählt werden.

Bei der Wahl der Stromstärke durch selektive Ansteuerung einzelner Elektrodenelemente ist als unterer Grenzwert die zur Aktivierung des motorischen Systems benötigte Stromstärke zu beachten. Als oberer Grenzwert sollte die Schmerzschwelle beachtet werden.

Die Elektrodenanordnung einer erfindungsgemäßen Vorrichtung läßt sich besonders einfach an den menschlichen Körper anlegen, wenn die Elektrodenelemente einer Elektrode auf einem gemeinsamen Träger aus elektrisch isolierendem Material angebracht sind. Dabei handelt es sich zweckmäßigerweise um einen bahnförmigen Träger aus textilem Material, wobei vorzugsweise jeder Elektrode ein eigener Träger zugeordnet ist, der die Anbringung der entsprechenden Elektrode unabhängig von der Anbringung anderer Elektroden ermöglicht. Im Rahmen der Erfindung ist dabei auch daran gedacht, die Träger der Elektroden, bspw. über einen Flächenhaftverschluß, an einem Hauptträger anzubringen und mit Hilfe dieses Hauptträgers an dem Körper zu fixieren. Der Hauptträger kann dabei bspw. in Form eines Gürtels aus einem flexiblen und/oder textilen Material verwirklicht sein.

Die Elektrodenfelder der erfindungsgemäßen Vorrichtung bilden die Schnittstelle, an der die elektrische Ladung von der Stimulationseinrichtung in die Haut eingeleitet bzw. daraus abgeleitet werden kann und sind vorzugsweise aus einem textilen Material oder einem anderen Material hergestellt, mit dem der elektrische Strom gemäß einem vorgegebenen Programm gleichmäßig über das Elektrodenfeld eingeleitet werden kann, so daß eine Muskelkontraktion hervorgerufen wird. Zweckmäßigerweise ist die Stimulationseinrichtung als Mehrkanaleinrichtung mit einer galvanischen Trennung zwischen den einzelnen Kanälen ausgeführt.

Die Stimulationseinrichtung umfaßt vorzugsweise Signalverbindungselemente zwischen der elektronischen Stimulationsanordnung und Steuereinrichtungen für die Elektrodenfelder, eine Befehlsschnittstelle, über die das Programm zur Aktivierung der Stimulation ausgewählt werden kann, so daß eine elektrische Aktivierung der Muskeln unter der erfindungsgemäßen Vorrichtung hervorgerufen wird, eine Befehlsschnittstelle zur drahtlosen Programmierung von einem geeigneten Computersystem (PC oder PDA), einen Eingang für Sensorsignale zur sensorsignalausgelösten elektrischen Stimulation, die Stimulationssequenzen an geeignete Muskelgruppen auslöst, wenn eine Bewegung erkannt wird, die zu Rückenbeschwerden führen kann. Mit der Stimulationseinrichtung einer erfindungsgemäßen Vorrichtung kann der elektrische Strom vorzugsweise intermittierend, kontinuierlich, moduliert, mit geringen oder hohen Frequenzen auf vier, sechs, acht oder mehr elektrisch unabhängig voneinander betreibbare Kanäle verteilt werden. Dabei wird zweckmäßigerweise eine bipolare Stimulation eingesetzt. Mit der Stimulationseinrichtung wird zweckmäßigerweise die Ladungsmenge pro Sekunde durch Änderung der Impulsdauer, der Intensität der Impulse und der Impulsfrequenz wahlweise eingestellt.

Bei einer besonders bevorzugten Ausführungsform der Erfindung ist die Stimulationseinrichtung zum Anlegen des elektrischen Stroms in Form von Stromimpulsen mit einer Frequenz im Bereich von 1 bis 100 kHz betreibbar, insbes. 1 bis 100 Hz, wobei eine Frequenz zwischen 20 und 50 Hz bevorzugt ist.

Bei einer Stimulationsfrequenz von 16 Impulsen pro Sekunde oder weniger wird ein unangenehmes Zittern erzielt, während bei einer Stimulationsfrequenz mit höheren Frequenzen eine Muskelanspannung erreicht wird. Je höher die Stimulationsfrequenz ist, desto angenehmer ist die Behandlung mit einer erfindungsgemäßen Vorrichtung für den Benutzer. Zur Zeit wird eine erfindungsgemäße Vorrichtung besonders bevorzugt mit einer Stimulationsfrequenz von 50 Impulsen pro Sekunde betrieben. Eine Stimulation mit mehr als 100 Hz hat keine anderen Effekte als eine Stimulation mit 100 Hz.

Wie vorstehend bereits erwähnt, ist es besonders zweckmäßig, wenn die Frequenz und/oder die Stromstärke und/oder die Phase und/oder die Spannung der Stromimpulse für mindestens zwei, vorzugsweise mindestens drei, insbes. vier oder mehr, Elektrodenpaare der Elektrodenanordnung einzeln oder in Gruppen getrennt voneinander einstellbar ist. Dabei kann die Stromstärke durch selektive Ansteuerung einzelner Elektrodenelemente und/oder einzelner Gruppen von Elektrodenelementen eines Elektrodenfeldes eingestellt werden. Insbesondere über die Phase können die Impulse beispielsweise bewußt chaotisch oder in Mustern miteinander koordiniert und/oder synchronisiert werden. Im Hinblick auf eine einfache Handhabung einer erfindungsgemäßen Vorrichtung ist die Elektrodenanordnung vorzugsweise lösbar an einem Gurt angebracht, der derart an den menschlichen Körper anlegbar ist, daß er den Körper im Lendenwirbelbereich umläuft, wobei die Elektrodenanordnung zwischen dem Gurt und dem Körper angeordnet ist. Dabei können die einzelnen Elektroden der Elektrodenanordnung auf ihrer dem Gurt zugewandten Seite isolierend ausgeführt sein.

Wie der vorstehenden Erläuterung erfindungsgemäßer Vorrichtungen zu entnehmen ist, ist ein damit betreibbares Verfahren zur transkutanen elektrischen Stimulation motorischer Nerven, bei dem ein elektrischer Strom über eine Elektrodenanordnung im Bereich der Rücken- und/oder Bauchmuskulatur an den menschlichen Körper angelegt wird, im wesentlichen dadurch gekennzeichnet, daß der elektrische Strom in Abhängigkeit von Körperbewegungen darstellenden Bewegungssignalen einer Bewegungssensoranordnung an die Elektrodenanordnung angelegt wird und/oder dadurch, daß vier oder mehr Muskelgruppen durch vier oder mehr Elektrodenpaare unabhängig voneinander aktiviert werden, wobei die Form und Größe der Stromeinleitungsstellen zweckmäßigerweise durch entsprechende Ansteuerung von einzelnen Elektrodenelementen einer als Elektrodenfeld ausgeführten Elektrode ausgewählt wird.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine schematische Darstellung einer erfindungsgemäßen Vorrichtung,
- **Fig. 2**: eine Darstellung einer an einem Gurt befestigten Elektrodenanordnung,
- **Fig. 3**: eine die selektive Ansteuerung einzelner Elektrodenelemente einer erfindungsgemäßen Elektrodenanordnung veranschaulichende Darstellung und
- **Fig. 4**: schematische Darstellungen zur Veranschaulichung gewünschter und schädlicher Bewegungsmuster.

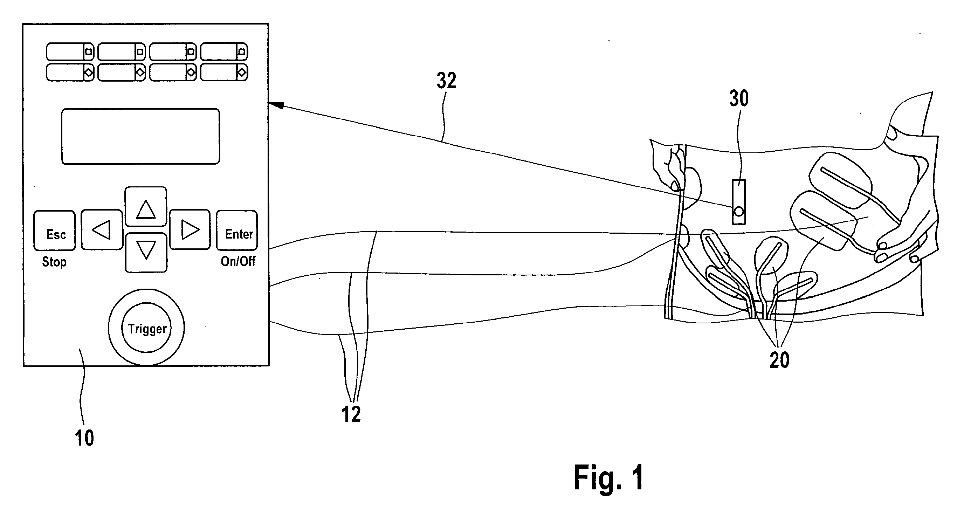

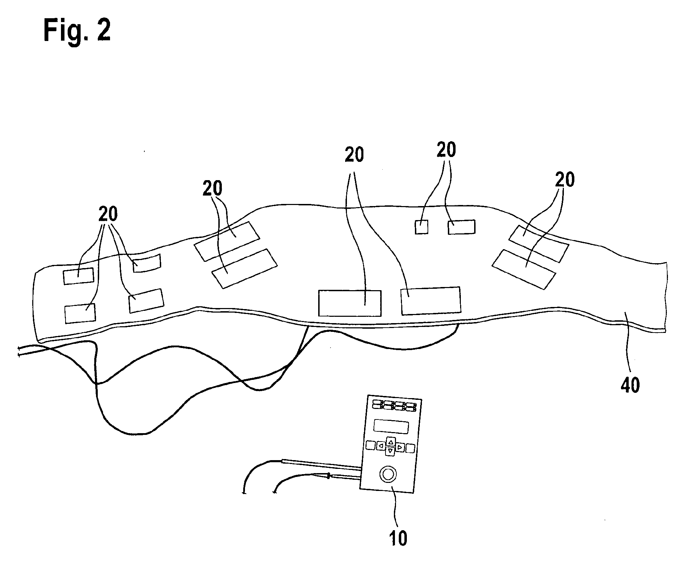

Die in Fig. 1 dargestellte Vorrichtung besteht im wesentlichen aus einer Steuereinrichtung 10, einer Mehrzahl von an einem Gurt 40 (vgl. Fig. 2) befestigten Elektrodenpaaren 20 und einem Sensorelement 30. Die Elektrodenpaare 20 werden gemäß Fig. 1 über Kabel 12 ausgehend von der Steuereinrichtung 10 angesteuert. Die dadurch oder anderweitig ausgelösten Bewegungen werden mit einer Bewegungssensoranordnung 30 erfaßt, die über ein Kabel 32 mit der Steuereinrichtung 10 verbunden ist, um so eine Regelung der Elektrodenansteuerung unter Verwendung der dadurch bewirkten Bewegungen als Regelgröße zu erreichen.

Wie in Fig. 2 zu erkennen ist, sind die Elektrodenpaare 20 an der dem Körper zugewandten Innenseite eines Gurts 40 angebracht.

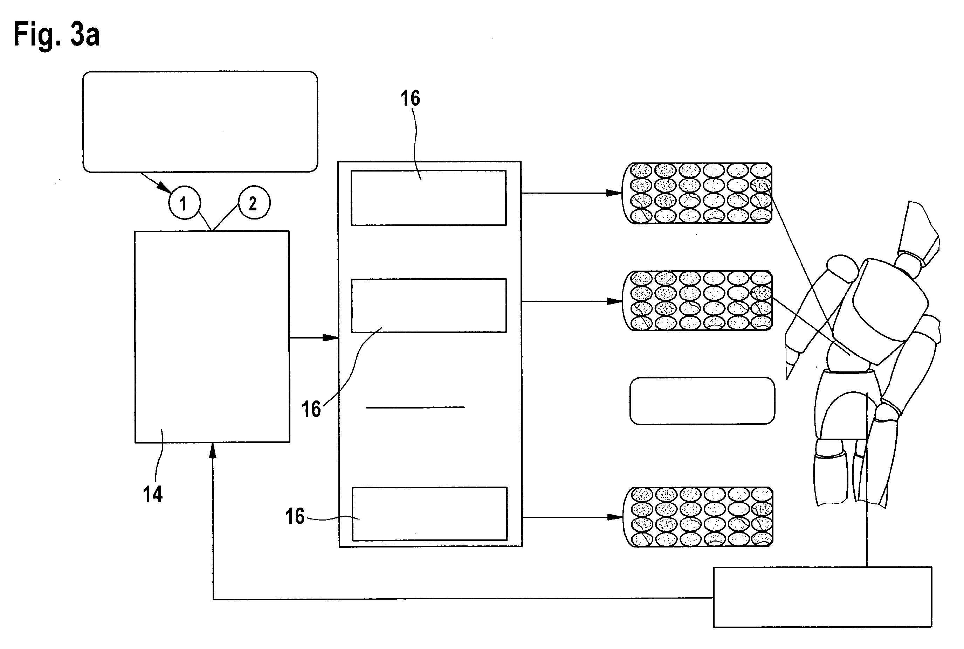

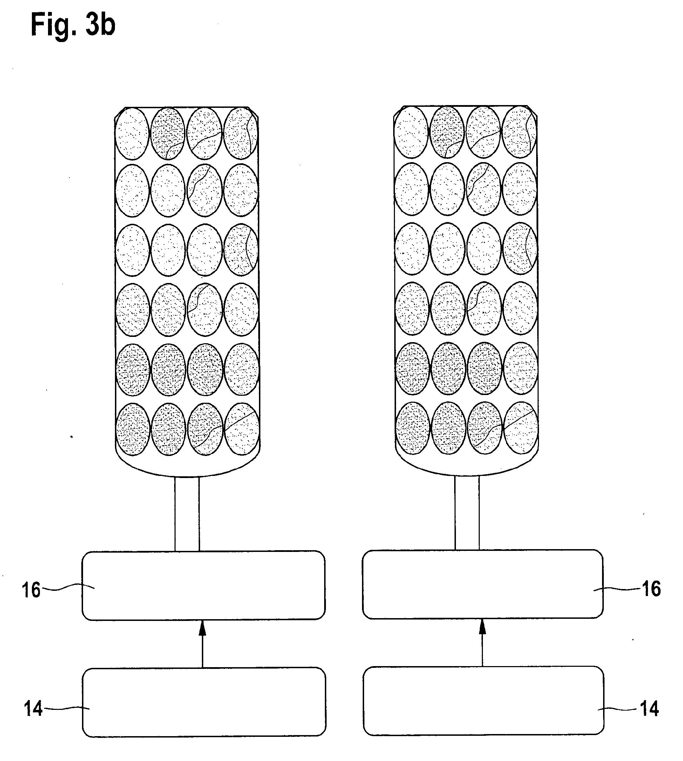

Gemäß Fig. 3 umfaßt die Steuereinrichtung 10 eine Mehrkanalstimulationseinrichtung 14 zur selektiven und unabhängigen Ansteuerung einzelner Elektrodenpaare sowie eine Mehrzahl von Elektrodensteuereinrichtungen 16. Mit der Mehrkanalstimulationseinrichtung wird für jeden einzelnen Kanal selektiv die Stimulationsfrequenz eingestellt, während mit den Elektrodensteuereinrichtungen für jedes Elektrodenpaar selektiv die Stromstärke dadurch eingestellt wird, daß einzelne Elektrodenelemente der in Form von Elektrodenfeldern gebildeten Elektroden angesteuert werden.

Wie besonders deutlich in Fig. 3b zu erkennen ist, sind die Elektrodenfelder in Form einer Rechteckmatrix mit einer Mehrzahl von Elektrodenelementzeilen (6 im dargestellten Beispiel) und Elektrodenelementspalten (4 im dargestellten Beispiel) angelegt, wobei die Elektrodenelemente jeder Elektrode an einem gemeinsamen, unabhängig von den Trägern der anderen Elektroden positionierbaren Träger festgelegt sind. Durch selektive Ansteuerung nur einzelner Elektrodenelemente der Elektrodenfelder kann die Stromstärke für die einzelnen Elektrodenpaare selektiv eingestellt werden, wobei durch geeignete Wahl der angesteuerten Elektrodenelemente auch der Ort der Stimulation eingestellt werden kann. Anders als im dargestellten Beispiel gezeigt, kann die Elektrodensteuereinrichtung auch in die Elektrodenfelder integriert sein oder in der Stimulationseinrichtung 14 integriert sein.

Mit den in der Zeichnung dargestellten Vorrichtungen können die einzelnen Elektrodenpaare auf Grundlage vorgegebener Programme angesteuert werden. Dabei liefert die Elektrodensteuereinrichtung 16 für jedes Elektrodenpaar selektiv einstellbare Impulssequenzen. Gemäß einer anderen Betriebsart können die Elektrodenpaare auf Grundlage von mit der Bewegungssensoranordnung 30 erfaßten Bewegungen des Benutzers angesteuert werden. Dabei werden mit der Bewegungssensoranordnung 30 schädliche Bewegungen erfaßt und einzelne Muskelgruppen so aktiviert, daß den schädlichen Bewegungen entgegengewirkt wird.

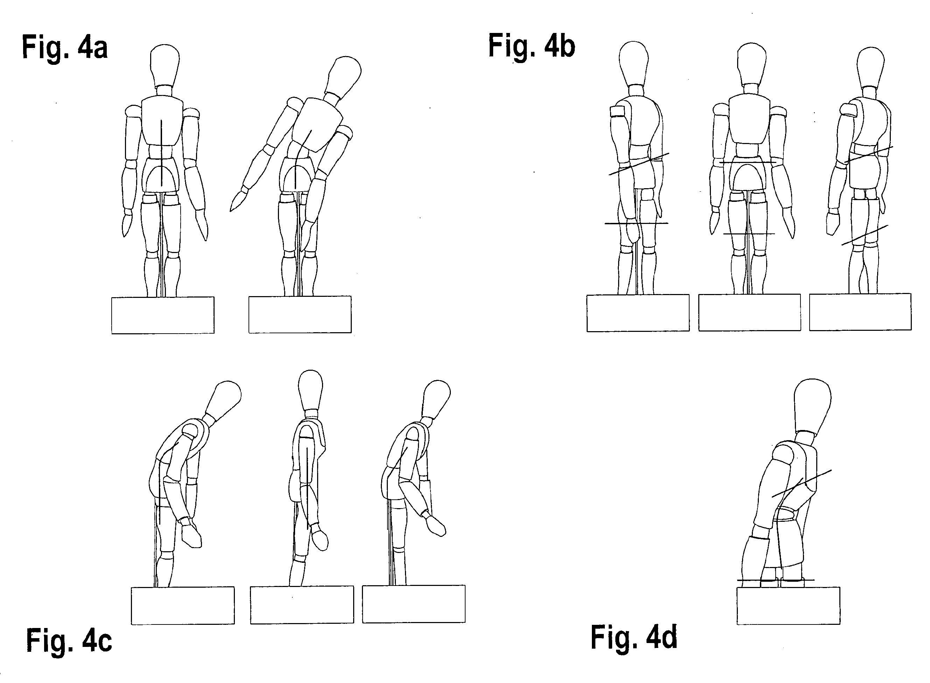

Gemäß Fig. 4a kann mit der Bewegungssensoranordnung 30 bspw. ein in Fig. 4a rechts dargestelltes seitliches Beugen des Oberkörpers als schädliche Bewegung erfaßt werden. Die korrekte Haltung ist in Fig. 4a links dargestellt.

Gemäß Fig. 4b kann mit der Bewegungssensoranordnung 30 auch eine in Fig. 4b links dargestellte Torsion des Rumpfes nachgewiesen werden und einzelne Muskelgruppen so angesteuert werden, daß anstelle der Torsion eine Drehbewegung der unteren Gliedmaßen hervorgerufen wird, wie in Fig. 4b mittig und rechts dargestellt.

Gemäß Fig. 4c kann mit der Bewegungssensoranordnung 30 auch eine schädliche Rumpfbeugung im Lendenwirbelbereich erfaßt werden und eine Ansteuerung einzelner Muskelgruppen so erfolgen, daß die Beugung im Lendenwirbelbereich durch eine Beugung im Hüftbereich ersetzt wird. Ferner ist es mit der Bewegungssensoranordnung 30 auch möglich, die in Fig. 4d dargestellte kombinierte Beuge- und Drehbewegung als schädliche Bewegung zu identifizieren und Muskelgruppen so anzusteuern, daß diese Bewegung vermieden wird.

Mit erfindungsgemäßen Vorrichtungen und Verfahren kann nicht nur ein gezielter Muskelaufbau erreicht werden, es können auch Rückenbeschwerden therapiert werden. Ferner kann eine Bewegungsschulung derart erfolgen, daß Rückenbeschwerden verursachende Bewegungen reduziert werden. Außerdem ist die Anwendung erfindungsgemäßer Vorrichtungen und Verfahren nicht auf den Bereich der Rücken- und Bauchmuskulatur beschränkt. Es können beliebige andere Muskelgruppen damit behandelt werden.

## Patentansprüche

1. Vorrichtung zur transkutanen elektrischen Stimulation motorischer und/oder sensorischer Nerven mit einer bspw. im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung, **dadurch gekennzeichnet, daß** der Stimulationseinrichtung mindestens eine zum Erfassen einer Körperbewegung betreibbare Bewegungssensoranordnung zugeordnet ist und die Stimulationseinrichtung zum Anlegen des elektrischen Stroms in Abhängigkeit von von der Bewegungssensoranordnung abgegebenen Bewegungssignalen betreibbar ist.

2. Vorrichtung nach Anspruch 1, **dadurch gekennzeichnet, daß** die Stimulationseinrichtung zum Vergleichen der mit dem mindestens einen Bewegungssensor erfaßten Körperbewegung mit einem vorgegebenen Bewegungsmuster betreibbar ist.

3. Vorrichtung zur transkutanen elektrischen Stimulation motorischer und/oder sensorischer Nerven, insbes. nach einem der vorhergehenden Ansprüche, mit einer bspw. im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung, **dadurch gekennzeichnet, daß** die Elektrodenanordnung vier oder mehr unabhängig voneinander mit elektrischem Strom beaufschlagbare Elektrodenpaare aufweist.

4. Vorrichtung zur transkutanen elektrischen Stimulation motorischer und/oder sensorischer Nerven, insbes. nach einem der vorhergehenden Ansprüche, mit einer bspw. im Bereich der Rücken- und/oder Bauchmuskulatur an den menschlichen Körper anlegbaren Elektrodenanordnung und einer zum Anlegen eines elektrischen Stroms an die Elektrodenanordnung betreibbaren Stimulationseinrichtung, bei der die Elektrodenanordnung mindestens ein Elektrodenpaar mit einer zum Einleiten des elektrischen Stroms in den menschlichen Körper über eine erste Kontaktfläche ausgelegten ersten Elektrode und einer zum Ableiten des elektrischen Stroms aus dem menschlichen Körper über eine zweite Kontaktfläche ausgelegten zweiten Elektrode aufweist, **dadurch gekennzeichnet, daß** die Größe, Form und/oder Position mindestens einer Kontaktfläche nach Anlegen des Elektrodenpaares an den menschlichen Körper veränderbar ist.

5. Vorrichtung nach Anspruch 4, **dadurch gekennzeichnet, daß** mindestens eine Elektrode ein Elektrodenfeld mit einer Anzahl von elektrisch voneinander isolierten und einzeln oder in Gruppen getrennt voneinander ansteuerbaren Elektrodenelementen mit einer Kontaktflächengröße von 1 mm² oder mehr aufweist.

6. Vorrichtung nach Anspruch 4 oder 5, **dadurch gekennzeichnet, daß** die gesamte Kontaktflächengröße des Elektrodenfeldes 1 cm² oder mehr beträgt.

7. Vorrichtung nach Anspruch 5 oder 6, **dadurch gekennzeichnet, daß** die Elektrodenelemente nach Art einer Matrix, insbes. einer Rechteckmatrix, in eine Anzahl von etwa parallel zueinander verlaufenden Reihen und eine Anzahl von etwa senkrecht zu den Reihen verlaufenden Spalten angeordnet sind.

8. Vorrichtung nach einem der Ansprüche 5 bis 7, **dadurch gekennzeichnet, daß** die Elektrodenelemente einer Elektrode auf einem gemeinsamen, insbes. bahnförmigen Träger aus elektrisch isolierendem Material angebracht sind.

9. Vorrichtung nach einem der vorhergehenden Ansprüche, **dadurch gekennzeichnet, daß** die Stimulationseinrichtung zum Anlegen des elektrischen Stroms in Form von Stromimpulsen mit einer Frequenz im Bereich von 1 bis 100 kHz, insbes. 1 bis 100 Hz, besonders bevorzugt 20 bis 50 Hz, betreibbar ist.

10. Vorrichtung nach Anspruch 9, **dadurch gekennzeichnet, daß** die Frequenz und/oder die Stromstärke und/oder die Phase und/oder die Spannung der Stromimpulse für mindestens zwei, vorzugsweise mindestens drei, insbes. vier oder mehr, Elektrodenpaare der Elektrodenanordnung einzeln oder in Gruppen getrennt voneinander einstellbar ist.

11. Vorrichtung nach Anspruch 10, **dadurch gekennzeichnet, daß** die Impulse miteinander koordinierbar und/oder synchronisierbar sind.

12. Vorrichtung nach Anspruch 9, **dadurch gekennzeichnet, daß** die Stromdichte durch selektive Ansteuerung einzelner Elektrodenelemente und/oder einzelner Gruppen von Elektrodenelementen eines Elektrodenfeldes einstellbar ist.

13. Vorrichtung nach einem der vorhergehenden Ansprüche, **dadurch gekennzeichnet, daß** die Elektrodenanordnung vorzugsweise lösbar an einem Gurt angebracht ist, der derart an den menschlichen Körper anlegbar ist, daß er den Körper im Lendenwirbelbereich umläuft, wobei die Elektrodenanordnung zwischen dem Gurt und dem Körper angeordnet ist.

14. Verfahren zur transkutanen elektrischen Stimulation motorischer und/oder sensorischer Nerven, bei dem ein elektrischer Strom über eine Elektrodenanordnung bspw. im Bereich der Rücken und/oder Bauchmuskulatur an den menschlichen Körper angelegt wird, **dadurch gekennzeichnet, daß** der elektrische Strom in Abhängigkeit von Körperbewegungen darstellenden Bewegungssignalen einer Bewegungssensoranordnung angelegt wird.
